(19) Europäisches Patentamt
European Patent Office
Office européen des brevets

(11) **EP 3 040 024 A1**

(12) **EUROPÄISCHE PATENTANMELDUNG**

(43) Veröffentlichungstag:
**06.07.2016 Patentblatt 2016/27**

(51) Int Cl.:
***A61B 5/12*** *(2006.01)*     ***A61B 5/00*** *(2006.01)*
***G10L 25/60*** *(2013.01)*

(21) Anmeldenummer: **15202918.7**

(22) Anmeldetag: **29.12.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **30.12.2014 AT 509502014**

(71) Anmelder: **Audio Lab Swiss AG**
**6312 Steinhausen (CH)**

(72) Erfinder:
• **DUPOR, Sanja**
**1230 Wien (AT)**
• **RIBIC, Zlatan**
**1230 Wien (AT)**

(74) Vertreter: **Babeluk, Michael**
**Florianigasse 26/3**
**1080 Wien (AT)**

(54) **VERFAHREN ZUR BESTIMMUNG DER QUALITÄT EINES ÜBERTRAGUNGSSYSTEMS**

(57) Die Erfindung betrifft ein Verfahren zur Bestimmung der Qualität eines Übertragungssystems in Hinblick auf die Sprachwahrnehmung, bei dem ein Kommunikationsindex berechnet wird, der ein Maß für die Verständlichkeit von Sprachinformation darstellt, indem einer Versuchsperson Testsignale angeboten werden, aus denen MCL (most comfortable level) und UCL (uncomfortable level) bestimmt werden. Eine Steigerung der Genauigkeit kann dadurch erreicht werden, dass die Testsignale auch Tonfolgen zur Bestimmung von zeitlichen Maskierungseffekten beinhalten, die in einem gemeinsamen Berechnungsschritt mit MCL und UCL zur Bestimmung des Kommunikationsindex berücksichtigt werden.

# Fig. 1

**Beschreibung**

Die Erfindung betrifft ein Verfahren zur Bestimmung der Qualität eines Übertragungssystems in Hinblick auf die Sprachwahrnehmung gemäß dem Oberbegriff von Patentanspruch 1.

Bei einem solchen Verfahren wird ein Kommunikationsindex berechnet, der ein Maß für die Verständlichkeit von Sprachinformation darstellt, indem einer Versuchsperson Testsignale angeboten werden, aus denen MCL (most comfortable level) und UCL (uncomfortable level) bestimmt werden.

Es ist bekannt, dass die Bestimmung der Hörschwelle von Personen keine zuverlässige Aussage darüber zulässt, inwieweit die betreffende Person in der Lage ist, Sprache akustisch wahrzunehmen und zu verstehen. Daher ist auch die Beurteilung von Übertragungssystemen, wie beispielsweise Hörgeräten, auf der Basis der Bestimmung der Hörschwelle keineswegs ausreichend.

Um diese Nachteile zu vermeiden, ist es bekannt geworden, Auswertungen auf der Basis von MCL und UCL vorzunehmen. Dabei werden eine Versuchsperson verschiedene Signale angeboten und es wird - abhängig von der Frequenz - einerseits ein Pegel bestimmt, der als besonders angenehm empfunden wird (MCL) und andererseits werden Pegel bestimmt, ab denen Signale als unangenehm laut empfunden werden (UCL). Die Differenz zwischen UCL und MCL wird hier als Toleranz T bezeichnet. Durch die Bestimmung von MCL und UCL lassen sich zusätzliche Informationen beispielsweise über das Hörvermögen einer Person im Hinblick auf das Sprachverständnis aber auch im Hinblick auf die Qualität eines Übertragungssystems ebenfalls Person im Hinblick auf das Sprachverständnis gewinnen. Eine solche Lösung ist beispielsweise in der TW 2011 43716 A beschrieben.

Es ist auch bekannt, dass das Sprachverständnis durch so genannte Maskierungseffekte in der Frequenz und in der Zeit beeinflusst wird. Dabei wird die Wahrnehmung von Signalen durch vorhergehende, gleichzeitige oder danach folgende Signale beeinflusst. Überlegungen dazu sind beispielsweise in der EP 31135 B oder der US 6,109,107 A offenbart.

Darüber hinaus sind direkte Verfahren entwickelt worden, die darauf abzielen, die Sprachwahrnehmung zu untersuchen. Dabei werden die Versuchspersonen beispielsweise mit einzelnen Wörtern in unterschiedlichen Lautstärken konfrontiert (Sprachaudiometrie). Solche Untersuchungen sind jedoch sehr unzuverlässig, da das Ergebnis beispielsweise sehr stark von der Muttersprache oder den allgemeinen Sprachkenntnissen der Versuchsperson abhängt. Auch sonstige intellektuelle Fähigkeiten der Versuchsperson gehen stark in das Ergebnis ein. Alternativ dazu werden auch sinnlose einsilbige Sprachfragmente (Logatome) zur Untersuchung verwendet. Auch damit können nur relativ unzuverlässige Ergebnisse erzielt werden.

Insgesamt ist es jedoch nicht in einfacher Weise möglich, das Wahrnehmungsvermögen für Sprache zu untersuchen. Insbesondere ist es bisher nicht möglich, zuverlässigen Untersuchungsverfahren anzugeben, die ohne Mitwirkung von besonders geschulten Personen stattfinden, die die erforderlichen Geräte bedienen und die Versuchspersonen untersuchen.

Aufgabe der vorliegenden Erfindung ist es, diese Nachteile zu vermeiden und eine Lösung anzugeben, die eine leichte Bedienbarkeit gewährleistet. Insbesondere sollen Verfahren und Vorrichtung angegeben werden, mit denen es möglich ist, Personen auch ohne Beisein von geschultem Personal zu untersuchen. Die Messung sollte dabei robust sein, insbesondere gegenüber den spezifischen sprachlichen Fähigkeiten und Kenntnissen der Versuchsperson.

Erfindungsgemäß werden diese Aufgaben durch die Merkmale von Patentanspruch 1 gelöst.

Dabei ist vorgesehen, dass die Testsignale auch Tonfolgen zur Bestimmung von zeitlichen Maskierungseffekten beinhalten, die in einem gemeinsamen Berechnungsschritt mit MCL und UCL zur Bestimmung des Kommunikationsindex berücksichtigt werden.

Das Phänomen der Maskierung kann in der Zeit oder in der Frequenz auftreten, hier ist die zeitliche Maskierung relevant (temporal resolution). Die bedeutet, dass Signale, die vor, während oder nach einem Störsignal übermittelt werden, schlechter wahrnehmbar sind, d.h. verdeckt werden.

Wesentlich an der vorliegenden Erfindung ist, dass die gemeinsame Berücksichtigung der beiden Aspekte eine wesentlich genauere und zuverlässigere Beurteilung des Sprachverständnisses ermöglicht. Dabei können insbesondere auch Zwischenergebnisse im Hinblick auf einen Aspekt in das Verfahren zur Bestimmung des anderen Aspekts einbezogen werden. Dies bedeutet beispielsweise, dass nach Bestimmung von MCL zeitliche Maskierungseffekte bei einem aus MCL direkt abgeleiteten Pegel, beispielsweise genau bei MCL gemessen werden.

Das erfindungsgemäße Verfahren ist nicht nur dazu geeignet, das natürliche Übertragungssystem, also das menschliche Ohr, zu untersuchen, sondern auch zwischengeschaltete technische Übertragungssysteme, wie etwa Hörgeräte oder Mobiltelefone, in Hinblick auf die Qualität bei der Sprachübertragung zu beurteilen.

Wesentlich an der vorliegenden Erfindung ist, dass aus der Kombination der Bestimmung von MCL und UCL und von zeitlichen Maskierungseffekten eine sehr zuverlässige Beurteilung der Kommunikationsqualität möglich ist, jedoch gleichzeitig das Messverfahren einfach gestaltet ist und ohne Mitwirkung hoch qualifizierter durchführbar ist.

MCL und UCL werden bestimmt, indem Signale, wie etwa Oktavenrauschen oder bei verschiedenen Pegeln angeboten werden und die Versuchspersonen angeben können, ob die Lautstärke angenehm, zu laut oder zu leise ist.

Es ist im Rahmen der Erfindung auch möglich, dass zur Bestimmung von MCL und UCL bandpassgefilterte Aufnahmen natürlicher Sprache verwendet werden. Der Vorteil dabei ist, dass Versuchspersonen die Lautstärke isolierter Töne nur schwer in zuverlässiger und reproduzierbarer Weise beurteilen können.

Bei der Sprachaudiometrie wird die Verständlichkeit in Abhängigkeit vom Schalldruck bestimmt. Da typischerweise die Messungen in Schritten von 5 dB für 20 Wörter oder mehr durchgeführt werden, sind die Messungen langwierig.

Da es wesentlich ist, MCL frequenzselektiv zu bestimmen, werden die Signale gefiltert, wobei die Bandbreite gerade groß genug gewählt wird, um die Natürlichkeit nicht allzu sehr zu beeinträchtigen, aber dennoch eine befriedigende Frequenzauflösung erreicht wird. Die Verständlichkeit kann so per se nicht gemessen werden, da gefilterte Sprache grundsätzlich schwer verständlich ist. Das Optimum der Verständlichkeit liegt bei etwa 1,6 kHz, ist aber dennoch nicht gut.

Zur Bestimmung von MCL und UCL können auch bandpassgefilterte speech like-Signale eingesetzt werden. Dabei handelt es sich um synthetische Signale, die strukturell natürlicher Sprache ähnlich sind. Da natürliche Sprache im Spektrum und in der Zeitdomäne stark fluktuiert, ist die Synthese solcher Signale schwierig.

Diese Untersuchung kann bei verschiedenen Frequenzen bzw. Frequenzbereichen durchgeführt werden, um die Aussagekraft zu erhöhen. Von besonderem Interesse bei der Auswertung ist die Toleranz, die sich aus

$$T = UCL - MCL \qquad (1)$$

ergibt. Je geringer diese Toleranz T ist, das heißt je näher MCL und UCL beieinander liegen, umso kritischer ist der Pegel und umso mehr ist das Sprachverständnis beeinträchtigt.

Werden die Messungen bei mehreren Frequenzen $f_1$, $f_2$, ... $f_n$ durchgeführt und somit die Werte $MCL(f_i)$ und $UCL(f_i)$ bestimmt, wird im Anschluss sinnvollerweise ein gewichteter Durchschnitt für die weitere Berechnung verwendet:

$$MCL = k_1 . MCL(f_1) + k_2 . MCL(f_2) + ... + k_n . MCL(f_n) \qquad (2)$$

bzw.

$$UCL = l_1 . UCL(f_1) + l_2 . UCL(f_2) + ... + l_n . U, CL(f_n) \qquad (3)$$

Wobei die Werte $k_i$ und $l_i$ für i = 1 ... n jeweils feste Gewichte darstellen.

Die Maskierungseffekte werden beispielsweise dadurch gemessen, dass ein Testsignal mit einer bestimmten Frequenz amplitudenmoduliert wird. Bei dem Testsignal kann es sich beispielsweise um ein schmalbandiges Rauschen, etwa ein Terzrauschen handeln. Bei der Messung wird ein Grenzwert für die Modulationsfrequenz bestimmt, ab dem das Rauschen nicht mehr intermittierend sondern durchgängig wahrgenommen wird.

Die Ursache dafür liegt in den Grenzdauern des Gehörs. Die typische Zeitkonstante für die Lautstärkeintegration beträgt etwa 200 ms. Signale, die kürzer sind, werden mit scheinbar verringerter Lautstärke wahrgenommen. In analoger Weise werden Amplitudenänderungen, die schneller als etwa 20 ms sind, nicht ganz wahrgenommen. Diese Zeitkonstante ist aber sehr wichtig, um Sprache zu verstehen. Leise und kurze Konsonanten sind für das Sprachverständnis wichtiger als laute und lange Vokale, können aber von Vokalen zeitlich maskiert werden.

Alternativ dazu können die Maskierungseffekte dadurch bestimmt werden, dass Testtöne vor, nach oder während einem maskierenden Signal angeboten werden und der Pegel und/oder die Zeitdauer bestimmt wird, ab dem der Testton wahrgenommen wird. Auch diese Untersuchungen können frequenzabhängig durchgeführt werden.

Die Frequenzabhängigkeit wird durch bandpassgefilterte Rauschsignale und einzelne Töne erzielt. Diese sind leicht zu unterscheiden. Sinnvollerweise wird dabei der Ton als Testsignal und das Rauschen als Maskierung verwendet. Beide werden in einer zeitlichen Abfolge angeboten, es geht beim Test darum, die Anwesenheit des Tons zu erfassen.

In einer besonders bevorzugten Ausführungsform des erfindungsgemäßen Verfahrens werden nicht nur die Messergebnisse der einzelnen Messungen miteinander kombiniert, sondern es werden auch Parameter der einzelnen Messungen von den Ergebnissen anderer Messungen abhängig gemacht. So kann etwa der Pegel des maskieren Signals bei einer bestimmten Frequenz aus den zuvor bestimmten Werten von MCL und UCL bei dieser Frequenz bestimmt werden. Dies führt zu genaueren Ergebnissen.

Als besonders vorteilhaft hat es sich herausgestellt, wenn die einzelnen Messungen für MCL/UCL bzw. die Maskierungseffekte alternierend durchgeführt werden. Auf diese Weise kann das Auftreten von Ermüdungseffekten wirksam unterdrückt werden.

Theoretisch ist es möglich, für die Bestimmung von MCL und UCL reine Töne bei einer bestimmten Frequenz zu verwenden. Es hat sich jedoch herausgestellt, dass wesentlich bessere Ergebnisse erzielt werden können, wenn anstelle dessen Realität nähere Signale verwendet werden. Die größte Realitätsnähe wird mit echten Sprachsignalen erreicht, wobei hier jedoch wiederum Störeffekte auftreten, die beispielsweise davon abhängen, wie gut die Versuchspersonen die für das Signal verwendete Sprache beherrscht. Auch die eindeutige Zuordnung von Frequenzen ist in diesem Fall nicht einfach. Schmalbandiges Rauschen hat sich in diesem Zusammenhang als besonders guter Kompromiss herausgestellt, da ein solches Signal eindeutig reproduzierbar und normierbar ist, aber dennoch ausreichende Nähe zur Realität aufweist. Der Frequenzbereich des Rauschens kann dabei eine Terz oder eine Quint sein. In manchen Fällen hat sich auch Oktavrauschen als günstig herausgestellt.

Die Erfindung betrifft auch eine Vorrichtung zur Bestimmung der Qualität eines Übertragungssystems in Hinblick auf die Sprachwahrnehmung, bei dem ein Kommunikationsindex berechnet wird, der ein Maß für die Verständlichkeit von Sprachinformation darstellt, mit einer Messeinrichtung zur Bestimmung von MCL (most comfortable level) und UCL (uncomfortable level).

Erfindungsgemäß ist vorgesehen, dass die Messeinrichtung auch Tonfolgen zur Bestimmung von zeitlichen Maskierungseffekten auswertet, und in einem gemeinsamen Berechnungsschritt mit MCL und UCL zur Bestimmung des Kommunikationsindex verwendet.

Mit einer solchen Vorrichtung ist es nicht nur möglich, natürliche Übertragungssysteme, wie das menschliche Ohr, sondern auch technische Übertragungssysteme, wie Hörgeräte, Telefongeräte oder dgl. in Hinblick auf die Sprachverständlichkeit zu untersuchen.

Parallel dazu werden Maskierungseffekte bestimmt. Dabei kann es sich um die minimale Zeitdifferenz Z handeln, ab der ein Testton nach einem maskierenden Signal von der Versuchsperson wahrgenommen werden kann.

Die zeitlichen Maskierungseffekte können beispielsweise mithilfe der Amplitudenmodulation festgestellt werden. Dazu wird ein Trägersignal CS mit einem Modulationssignal AM multipliziert, dass unter idealen Bedingungen Werte zwischen 0 und 2 annehmen kann dabei muss die Frequenz $f_m$ des Modulationssignals viel kleiner als die Trägerfrequenz $f_c$ sein ($f_c \gg f_m$).

Bei "voller" Modulation ändert sich somit der Ausgangswert zwischen 0 und 2.

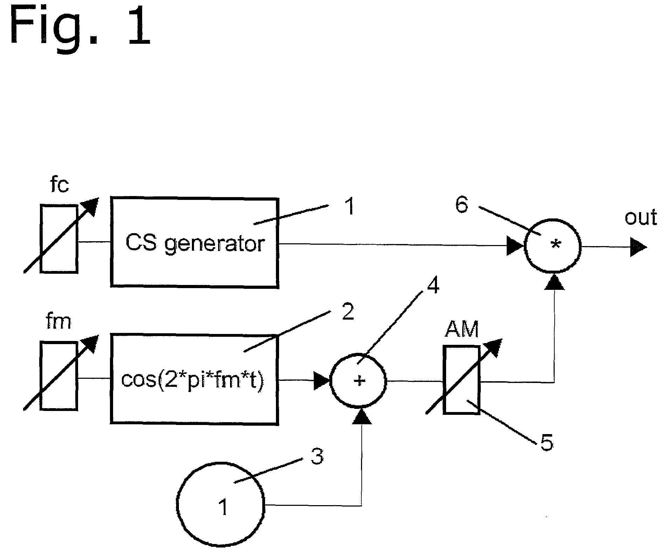

In der Fig. 1 ist die grundsätzliche Erzeugung des modulierten Signals dargestellt. Der Regler AM dient dazu, die Modulationstiefe einzustellen, so dass der Ausgangswert zwischen a und b liegt, mit

$$0 < a < b < 2.$$

In an sich bekannter Weise entstehen bei der Amplitudenmodulation so genannte Seitenbänder mit Frequenzen von $f_c - f_m$ und $f_c + f_m$.

Es hat sich herausgestellt, dass die zeitliche Maskierung am besten zu messen ist, wenn die Trägerfrequenzen $f_c$ oberhalb von etwa 500 Hz liegen und die Modulationsfrequenzen maximal 100 Hz betragen. Dadurch ist die obige Bedingung $f_c \gg f_m$ erfüllt.

Die Frequenzen für die Modulation sind regelbar. Bei der Untersuchung kann beispielsweise bei einer vorgegebenen Modulationstiefe mit sehr kleinen Modulationsfrequenzen $f_m$ = 1Hz begonnen werden. Danach wird $f_m$ so lange erhöht, bis der Proband die Schwankungen der Lautstärke nicht mehr wahrnehmen kann. Wenn dabei allerdings ein Ton, also ein Sinussignal als Träger verwendet wird, dann stört die Wahrnehmung der Seitenbänder, wodurch die Anwendung des Verfahrens stark beeinträchtigt wird. Daher wird bevorzugt schmalbandiges Rauschen als Träger eingesetzt. Dieses wird beispielsweise dadurch erzeugt, dass weißes Rauschen mit einem Hochpass- und einem Tiefpassfilter behandelt wird. Die Ordnungen dieser in Serie geschalteten Filter muss nicht gleich sein. Die Bandbreite des Signals sollte nicht zu klein sein, um die oben erläuterte Störung durch Seitenbänder zu vermeiden. Bandbreiten, wie Oktav, Quint oder Terz haben sich bewährt.

Es ist auch möglich, die Maskierung auf andere Art zu messen. Dabei wird in einer sich wiederholenden Sequenz zuerst ein Rauschen und danach ein Tonburst angeboten, der zeitlich begrenzt ist. Aus den Einstellparametern kann auf die Maskierung rückgeschlossen werden.

In der Folge werden bevorzugte Ausführungsvarianten der Erfindung unter Bezugnahme auf die beigefügten Figuren näher erläutert. Es zeigen:

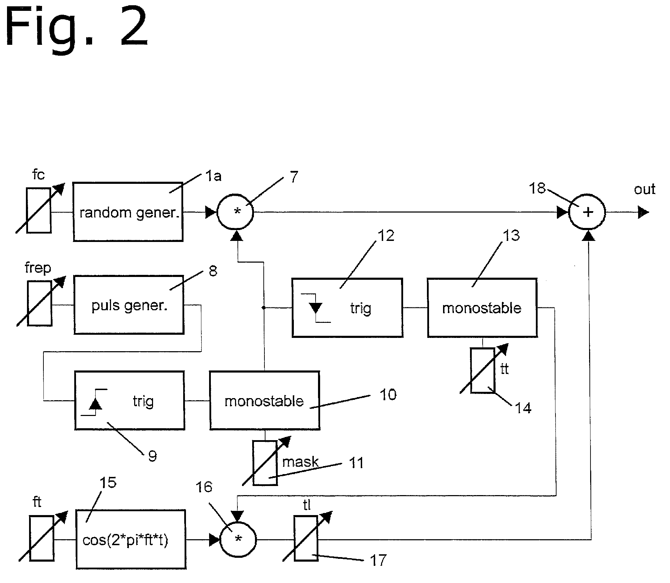

Fig. 1 und Fig. 2 zwei unterschiedliche Ausführungsvarianten zur Bestimmung der Maskierung in Schaltungsdiagrammen.

Bei der Ausführungsvariante von Fig. 1 wird in einem Signalgenerator 1 das Trägersignal CS beispielsweise in Form eines schmalbandigen Rauschens mit einstellbarer Mittelfrequenz $f_c$ erzeugt. Als Modulationssignal wird in einem Sinusgenerator 2 das Modulationssignal mit einstellbarer Frequenz $f_m$ erzeugt. Die in 3 erzeugte Konstante 1 wird im Addierer 4 zum Sinussignal aus 2 addiert und in einem Regler 5 komprimiert, um die gewünschte Modulationstiefe einzustellen. Das daraus resultierende Signal wird im Multiplikator 6 mit dem im Signalgenerator 1 erzeugten Trägersignal CS multiplikativ verknüpft. Dieses Signal wird der Versuchsperson angeboten.

Die Modulationsfrequenz $f_m$, bei der die Versuchsperson das Signal gerade noch als an- und abschwellend wahrnehmen kann, ist ein Maß für die zeitlichen Maskierungseffekte. Je höher diese Frequenz ist, desto geringer ist die Maskierung. Selbstverständlich hängt diese Grenzfrequenz auch von der Trägerfrequenz $f_c$ und der Modulationstiefe ab. Daher können Messungen mit unterschiedlichen Parameterkonstellationen durchgeführt werden, die insgesamt eine robustere Aussage über die Maskierung ermöglichen.

Bei der Ausführungsvariante von Fig. 2 wird im Signalgenerator 1a ein Rauschen mit einstellbarer Mittelfrequenz $f_c$ erzeugt und einem Multiplikator 7 zugeführt.

In einem Pulsgenerator 8 wird mit einer einstellbaren Wiederholfrequenz $f_{rep}$ ein Trigger 9 angesteuert, um im Glied 10 einen Impuls mit einer durch einen Regler 11 einstellbaren Dauer mask zu erzeugen. Dieser Impuls wird ebenfalls dem Multiplikator zugeführt.

Gleichzeitig wird dieses Signal als Auslöser für einen weiteren Trigger 12 verwendet, der im Glied 13 einen Impuls mit einer durch den Regler 14 einstellbaren Dauer tt erzeugt. Während dieses Impulses wird ein Sinussignal, das in einem Sinusgenerator 15 erzeugt wird, über den Multiplikator 16 und einen Regler 17 einem Addierer 18 zugeführt, der das Untersuchungssignal herstellt.

Die Funktion dieser Schaltung kann folgendermaßen zusammengefasst werden:

Am Beginn jeder Periode wird ein Rauschen erzeugt, das während der Zeitdauer mask übertragen wird. Unmittelbar danach wird für die weitere Zeitdauer tt ein Sinussignal mit einer einstellbaren Amplitude übertragen. Danach folgt eine Pause, bis der Vorgang erneut beginnt. Die Länge der Pause ist $1/f_{rep}$ -mask - tt.

Der Test besteht darin, abzufragen, ab wann der Sinuston nach dem Rauschen wahrgenommen wird. Dies hängt von der Dauer und der Lautstärke ab. Auf diese Weise kann für verschiedene Bedingungen ein Wert für Z gewonnen werden, der gegebenenfalls noch normiert werden kann, um vergleichbar zu sein.

Letztlich wird ein Kommunikationsindex KI nach einer allgemeinen Formel bestimmt:

$$KI = f(MCL, UCL, Z) \qquad\qquad (4)$$

Wenn man die Toleranz T gemäß Formel (1) einsetzt, kann man die Formel (4) spezifizieren:

$$KI = g(T) / h(Z) \qquad\qquad (5)$$

Wobei g und h Funktionen von T bzw. Z sind, die grundsätzlich mit wachsendem Argument ansteigen und vorzugsweise monoton steigend sind.

Zusätzlich dazu können in die Funktion KI der Gleichung (5) auch MCL und/oder UCL direkt eingehen, dies aber im Wesentlichen in der Art von Korrekturgrößen.

Ein Beispiel für eine solche Gleichung ist konkret:

$$KI = (a \cdot T^b + c) / ((MCL - d)^e + h \cdot Z^i + j) \qquad\qquad (6)$$

mit a, b, c, d, e, h, i, j mit empirisch bestimmten Koeffizienten. Dabei sind a, b, e, h und i positiv.

**Patentansprüche**

1. Verfahren zur Bestimmung der Qualität eines Übertragungssystems in Hinblick auf die Sprachwahrnehmung, bei dem ein Kommunikationsindex berechnet wird, der ein Maß für die Verständlichkeit von Sprachinformation darstellt, indem einer Versuchsperson Testsignale angeboten werden, aus denen MCL (most comfortable level) und UCL (uncomfortable level) bestimmt werden, **dadurch gekennzeichnet, dass** die Testsignale auch Tonfolgen zur Bestimmung von zeitlichen Maskierungseffekten beinhalten, die in einem gemeinsamen Berechnungsschritt mit MCL und UCL zur Bestimmung des Kommunikationsindex berücksichtigt werden.

**2.** Verfahren nach Anspruch 1, **dadurch gekennzeichnet, dass** die Testsignale zur Bestimmung der zeitlichen Maskierungseffekte einen Pegel aufweisen, der im Wesentlichen MCL entspricht.

**3.** Verfahren nach einem der Ansprüche 1 oder 2, **dadurch gekennzeichnet, dass** die Signale zur Bestimmung von MCL und UCL einerseits und zur Bestimmung von Maskierungseffekten andererseits in zeitlicher Abfolge abwechselnd angeboten werden.

**4.** Verfahren nach einem der Ansprüche 1 bis 3, **dadurch gekennzeichnet, dass** sowohl zur Bestimmung von MCL und UCL als auch zur Bestimmung von Maskierungseffekten schmalbandiges Rauschen vorgesehen ist.

**5.** Verfahren nach einem der Ansprüche 1 bis 4, **dadurch gekennzeichnet, dass** in die Berechnung eine Potenz der Toleranz T eingeht, die als Differenz von UCL und MCL definiert ist.

**6.** Verfahren nach einem der Ansprüche 1 bis 5, **dadurch gekennzeichnet, dass** zur Bestimmung von MCL und UCL bandpassgefilterte Aufnahmen natürlicher Sprache verwendet werden.

**7.** Verfahren nach einem der Ansprüche 1 bis 5, **dadurch gekennzeichnet, dass** zur Bestimmung von MCL und UCL bandpassgefilterte speech like-Signale verwendet werden.

**8.** Verfahren nach einem der Ansprüche 1 bis 7, **dadurch gekennzeichnet, dass** die zeitliche Maskierung durch das Anbieten amplitudenmodulierter Signale bestimmt wird.

**9.** Verfahren nach einem der Ansprüche 1 bis 8, **dadurch gekennzeichnet, dass** der Kommunikationsindex aus einem Quotienten einer monoton von der Toleranz, das ist die Differenz von UCL und MCL abhängenden Größe, und einer monoton von einer Zeitmaskierungskonstanten Z bestimmt wird.

**10.** Vorrichtung zur Bestimmung der Qualität eines Übertragungssystems in Hinblick auf die Sprachwahrnehmung, bei dem ein Kommunikationsindex berechnet wird, der ein Maß für die Verständlichkeit von Sprachinformation darstellt, mit einer Messeinrichtung zur Bestimmung von MCL (most comfortable level) und UCL (uncomfortable level), **dadurch gekennzeichnet, dass** die Messeinrichtung auch Tonfolgen zur Bestimmung von zeitlichen Maskierungseffekten auswertet, und in einem gemeinsamen Berechnungsschritt mit MCL und UCL zur Bestimmung des Kommunikationsindex verwendet.

**11.** Vorrichtung nach Anspruch 10, **dadurch gekennzeichnet, dass** als Übertragungssystem ein Kopfhörer vorgesehen ist, der dazu bestimmt ist, von einer Versuchsperson getragen zu werden.

**12.** Vorrichtung einem der Ansprüche 10 oder 11, **dadurch gekennzeichnet, dass** das Übertragungssystem ein Hörgerät umfasst.

# Fig. 1

# Fig. 2

Europäisches
Patentamt

European
Patent Office

Office européen
des brevets

## EUROPÄISCHER RECHERCHENBERICHT

Nummer der Anmeldung

EP 15 20 2918

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>A | US 2014/148724 A1 (UNGSTRUP MICHAEL [DK] ET AL) 29. Mai 2014 (2014-05-29)<br>* Absätze [0035], [0052] - [0068]; Ansprüche; Abbildungen *<br>----- | 1-4,6-8, 10-12<br>5,9 | INV.<br>A61B5/12<br>A61B5/00<br>G10L25/60 |
| A | EP 2 226 794 A1 (HARMAN BECKER AUTOMOTIVE SYS [DE]) 8. September 2010 (2010-09-08)<br>* das ganze Dokument *<br>----- | 1-12 | |
| A | US 2011/313315 A1 (ATTIAS JOSEPH [IL] ET AL) 22. Dezember 2011 (2011-12-22)<br>* das ganze Dokument *<br>----- | 1-12 | |
| A | EP 0 661 905 A2 (PHONAK AG [CH])<br>5. Juli 1995 (1995-07-05)<br>* das ganze Dokument *<br>----- | 1-12 | |

RECHERCHIERTE
SACHGEBIETE (IPC)

A61B
G10L
H04R
A61F
G02C
H03H
G10K

Der vorliegende Recherchenbericht wurde für alle Patentansprüche erstellt

| Recherchenort | Abschlußdatum der Recherche | Prüfer |
|---|---|---|
| Den Haag | 25. April 2016 | Crisan, Carmen-Clara |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 15 20 2918

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-04-2016

| Im Recherchenbericht angeführtes Patentdokument | Datum der Veröffentlichung | Mitglied(er) der Patentfamilie | Datum der Veröffentlichung |
|---|---|---|---|
| US 2014148724 A1 | 29-05-2014 | EP 2740279 A1<br>US 2014148724 A1<br>WO 2013017169 A1 | 11-06-2014<br>29-05-2014<br>07-02-2013 |
| EP 2226794 A1 | 08-09-2010 | EP 2226794 A1<br>JP 5439200 B2<br>JP 2010211190 A<br>US 2010226501 A1 | 08-09-2010<br>12-03-2014<br>24-09-2010<br>09-09-2010 |
| US 2011313315 A1 | 22-12-2011 | US 2011313315 A1<br>WO 2010092566 A1 | 22-12-2011<br>19-08-2010 |
| EP 0661905 A2 | 05-07-1995 | AT 229729 T<br>DE 59510501 D1<br>DK 0661905 T3<br>EP 0661905 A2<br>EP 1207718 A2 | 15-12-2002<br>23-01-2003<br>07-04-2003<br>05-07-1995<br>22-05-2002 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- TW 201143716 A
- EP 31135 B
- US 6109107 A